Europäisches Patentamt

European Patent Office

Office européen des brevets

(19)

(11) Publication number: **0 089 830**
**B1**

(12) **EUROPEAN PATENT SPECIFICATION**

(45) Date of publication of patent specification: **28.05.86**

(21) Application number: **83301523.3**

(22) Date of filing: **18.03.83**

(51) Int. Cl.⁴: **C 01 B 5/00, B 01 J 35/00, C 12 M 1/04, B 01 J 23/40**

(54) Deoxygenation process.

(30) Priority: **19.03.82 GB 8208090**

(43) Date of publication of application:
**28.09.83 Bulletin 83/39**

(45) Publication of the grant of the patent:
**28.05.86 Bulletin 86/22**

(84) Designated Contracting States:
**BE CH DE FR GB IT LI NL SE**

(56) References cited:
**GB-A- 882 715**
**GB-A-1 277 625**
**GB-A-1 567 900**
**US-A-3 258 360**
**US-A-4 078 893**

**CHEMICAL ABSTRACTS, vol. 91, no. 24, December 1979, page 119, no. 195265z, Columbus, Ohio, USA**

(73) Proprietor: **JOHNSON MATTHEY PUBLIC LIMITED COMPANY**
**43 Hatton Garden**
**London, EC1N 8EE (GB)**

(72) Inventor: **King, Frank**
**Curzon House South Stoke Road**
**Woodcote Reading Berkshire (GB)**
Inventor: **Thomson, Alasdair Iain**
**64 Kidmore Road Caversham**
**Reading Berkshire (GB)**

(74) Representative: **Arthur, Bryan Edward et al**
**4 Dyers Buildings Holborn**
**London, EC1N 2JT (GB)**

Courier Press, Leamington Spa, England.

# 0 089 830

**Description**

This invention relates to a deoxygenation process in which oxygen is catalytically reacted with hydrogen to produce water. The invention is particularly useful for reducing the oxygen concentration in a sealed container such as an anaerobic jar.

One application of a deoxygenation process is in the formation of an anaerobic atmosphere for the culture of anaerobic microorganisms. Such cultures are generally carried out in an anaeorbic jar. Recent versions of such jars typically comprise a closeable and sealable container provided with means for the introduction of gaseous hydrogen and carbon dioxide, a catalyst being located within the jar. In use, the jar is loaded with dishes containing the microorganisms and the culture growth medium and is then closed and sealed against the surrounding atmosphere. Gaseous hydrogen and carbon dioxide are introduced, either from an external source or by *in situ* chemical generation. The oxygen present in the jar reacts with the hydrogen on the catalyst surface to produce water vapour. The introduced carbon dioxide maintains the pressure inside the jar at approximately atmospheric, suitable safety ad non-return valves being provided.

Anaerobic microorganisms such as bacteria reproduce only in atmospheres containing less than about 2% oxygen and it is a requirement of anaerobic jars that this oxygen level be obtained within 2 hours of sealing the jar. Catalysts which have been used to achieve this comprise palladium but the catalytic reaction is highly exothermic. Because of this and because the mixing of hydrogen and oxygen results in temporary formation of mixtures thereof which exceed the explosive limit, there is a risk of explosion occurring. One catalyst system used to overcome this hazard is described in published European patent specification No. 0,002,967, in which the palladium is disposed on alumina pellets held loose in and throughout the holes and folds of a folded or rolled up metal foil net which is located inside a container having holes for the inflow and outflow of gases. The metal foil net acts to dissipate localised hot spots and to prevent any catalyst "fines", produced largely as a result of the damaging thermal gradients created by the hot spots, from escaping into the potentially explosive gas mixture. The container, which is preferably of gauze woven from a heat-resistant and heat-conducting materials, effectively acts as a flame trap. Although such a catalyst typically contains about 30 mg of palladium, it is nevertheless, in theory, economical to use since the catalytic activity is regenerable by heating in *vacuo* and the system may therefore be re-used. Up to 30 such regenerations may be successfully achieved, but the catalysts are susceptible to poisoning, for example by $H_2S$ evolved by certain bacteria. The catalysts may in consequence suffer a marked drop in activity, if not a complete loss, well before this number of regenerations have been carried out. This is a particularly acute practical disadvantage because there is no way of determining, following a regeneration and prior to the next use, whether or not the regeneration has been successful. Such catalysts are therefore remarkably uneconomic to use in that, for reliable activity, they have to be discarded and replaced well before their maximum life expectancy is realised.

In an attempt to overcome these disadvantages, a so-called "disposable" catalyst has been proposed. Such a catalyst comprised a very small amount (typically less than 5 mg) of palladium on alumina disposed directly on a corrugated, heat-dissipating substrate. Catalyst accessibility to reactants was excellent and activity therefore assured. Conveniently, when the hydrogen and carbon dioxide were generated *in situ,* the substrate comprised the outside of the metal foil pack containing the generator chemicals. The spent generator pack and catalyst could thus readily be discarded following use. This proposal proved to be unacceptable in use, however, because particles of palladium on alumina became easily loosened during handling and due to the effect of thermal gradients, and dropped off the substrate as "fines". This creates a risk of explosion in the hydrogen/oxygen mixture.

It is an object of the present invention to provide a deoxygenation process by catalytic reaction of hydrogen and oxygen especially in an anaerobic jar, which renders possible the use of the concept of a disposable catalyst, or which provides a catalyst which is more reliably regenerable than prior art catalysts.

The present invention accordingly provides a deoxygenation process which comprises reacting together hydrogen and oxygen over a catalyst, wherein the catalyst comprises palladium, platinum, rhodium and/or iridium disposed on a porous activated carbon, fibrous woven cloth support having an average surface area in the range 200 to 1200 $m^2g^{-1}$.

The process of the invention is particularly useful for removing oxygen from a sealed container such as an anaerobic jar, but may also be used for, for example, removing the final traces of oxygen from hydrogen stored under pressure in a cylinder.

In the catalyst, the metals palladium, platinum, rhodium and iridium, which may initially be present either in the metallic state or as their oxides or salts, initiate the deoxygenation process of at room temperature. They will hereinafter be referred to as "catalytic metal" or "metal". The choice of which particular catalytic metal to use will be decided largely on economic grounds and usually palladium and/or platinum will be preferred.

The material of the carbonaceous support comprises activated carbon. We have found that this material is particularly useful as catalyst supports for use in deoxygenation processes due to its affinity for the catalytic metal and its high heat conductivity. The high affinity for the metal results in the metal being very strongly bound to the support. The high heat conductivity facilitates dissipation of heat generated at the catalyst, thus avoiding the formation of hot·spots and "fines", while at the same time maintaining a

2

sufficiently high overall temperature to reduce the tendency of the catalyst to become "flooded" with condensed water of reaction. The avoidance of hot spots and "fines" are safety features. In addition, a carbonaceous catalyst support generally (depending on its mode of preparation) has a spread of active surface groups which provide for good dispersion, as well as binding, of the metal, compared with dispersion on alumina and other refractory oxide supports. Furthermore, a carbonaceous support has absorbent properties which enable it to absorb any hydrogen sulphide or other catalyst-poisoning molecules, thus guarding the metal against poisoning.

Woven carbonaceous cloths have all the advantages of carbonaceous supports in general with the particular further advantage of high strength. This contributes, together with the high heat conductivity possessed by carbonaceous supports in general, to excellent resistance to production of "fines". The precautions required to prevent "fines" in the hydrogen/oxygen atmosphere can therefore be minimised. Additionally, they have a relatively open structure, which aids diffusion of reactants, and can also be moulded or folded as well as cut to shape. We have found that a catalyst comprising catalytic metal disposed on a carbonaceous cloth support can for most purposes conveniently and safely be contained without further support directly in a heat-conductive gauze or other perforate container which acts as a flame trap.

Carbonaceous cloth is obtainable in the form of activated carbon or graphite, and is generally formed by carbonising or graphitising a textile cloth, which may be in piece or ribbon form. Naturally-occurring or synthetic textiles such as viscose rayon may be used as starting material, synthetic textiles being preferred for their greater strength. Carbonaceous cloths have high flexibility, coherence and tensile strength compared with other forms of carbonaceous material, and provide for a very high degree of dispersion of catalytic metal on and within the pores thereof. We have found that the average surface area of carbon cloths should be within the range 200—1200 $m^2.g^{-1}$ for good dispersion and catalytic activity. Ideally the range should be 400 to 1000 $m^2 g^{-1}$ to avoid or reduce the incidence of "flooding" of the support by water of reaction, which tends to occur in supports having higher surface areas and hence more micropores.

The thickness of woven carbonaceous cloths typically varies between about 0.3 to 2.0 mm. We have found that yarn having a diameter of about 0.5 mm and woven to about 12 threads per cm provides a suitable support for a deoxygenation catalyst although the dimensions of the yarn and resulting woven cloth do not appear to be critical on the effectiveness of the catalyst in the deoxygenation process.

The composition of carbonaceous cloths depends on whether the cloth comprises activated carbon or graphite. An activated carbon cloth typically contains 92 to 97% carbon, 0.2 to 1% ash and 2 to 7% volatiles, whereas a graphite cloth typically contains 99% or more carbon, up to 0.2% ash and up to 1% volatiles, all percentages being by weight. Trace quantities of base metals, for example lead, zinc and calcium, may be present, generally as their salts such as the chloride.

The carbon supports used, may be activated by any of the known activation procedures such as steam activation or by treating with phosphoric acid or hydrogen peroxide.

Suitable cloth supports are known as "C-tex" (an activated carbon cloth available from Siebe Gorman & Co Ltd) and as "TCM" "TGM" grades of cloth (respectively carbon and graphite cloths) available from Le Carbone (Gr Britain) Ltd. "RCM" and "RGM" grades of ribbon (respectively carbon and graphite) are also available from the same supplier. Another form is available as "Sigratex" SP woven fabric from Sigri Elektrographit GmbH. Yet a further form is available from Charcoal Cloth Ltd.

The catalytic metal is disposed on and within the pores of the support as a dispersion of crystallites for most purposes having a maximum metal content at the surface of the support and a gradual decline in metal content towards the interior. Such catalysts and the methods by which they may be prepared are known in the catalyst art as "surface-impregnated catalysts" and "surface impregnation techniques" respectively. The depth of impregnation cannot be generally specified since it depends on such variables as the porosity of the support and the method of disposing the catalytic metal.

The catalytic metal is present either alone or as a mixture or alloy with another platinum group or base metal as promoter or extender. Palladium, platinum, rhodium and iridium generally have the property of initiating the deoxygenation process at room temperature; these may be used alone or as mixtures or alloys of two or more thereof, optionally with a minor quantity (typically 10% or less) of other platinum group metal or base metal as promoter and/or extender. Metals which may be used in this way include ruthenium, nickel and silver. The term "alloy" does not imply a uniform composition throughout the metallic phase but rather bi-metallic or multi-metallic clusters in which the ratios of the component metals in the individual crystallites have a range the mean of which corresponds to the overall composition.

The catalytic metal or metals may be present initially either as their oxides or in the zero-valent state. In the former case, they are reduced to the zero-valent state under the conditions of the deoxygenation process.

The catalytic metal is preferably present as a dispersion of crystallites having a metal area within the range 3—100 $m^2 g^{-1}$ of catalyst. Although, for optimum catalytic activity, the particle size should be as low as possible, and hence the metal area as high as possible, we have found that the average particle size range is usually from 2 to 7.5 nm inclusive, or even in the narrower range 3 to 5 nm inclusive. By the expression "average particle size range", we mean a range within which the average of all the particles present will fall. Metal area is dependent however on metal loading as well as particles size.

The metal loading of the catalytic metal present on the support can be as high as 10% w/w but is

3

preferably lower. For reasons of economics, the loading should be as low as possible, provided that the desired catalytic activity is achieved. The minimum metal loading for a given catalyst depends, *inter alia,* on the nature and properties of the carbonaceous support. For example, since activated charcoal promotes better dispersion of metal than does graphite, an activated carbon cloth support may require only up to 2.5 wt% inclusive of catalytic metal whereas a graphite cloth may require up to 5 wt% inclusive of catalytic metal. Pellets would also require a higher loading than cloth, particularly than an activated carbon cloth. A further factor relating to metal loading and metal area is that the trace metals which may be present in the carbon may have a poisoning influence on the catalyst. Sufficient catalytic metal is required to overcome the poisoning influence and thus provide the necessary catalytic effectiveness. We have found in practice that an effective catalyst comprises from 1.5 to 2 mg palladium disposed on a square of activated carbon cloth having a side measurement of 2.5 cm, equivalent to 1.67—2.2% by weight of palladium.

The low catalytic metal loading required by a carbonaceous cloth support and especially by an activated carbon cloth support so improves the economics of the deoxygenation process over the economics using prior art catalysts that a catalyst using a cloth support can be discarded after only one use in a deoxygenation process, for example in an anaerobic jar. Thus a fresh catalyst can economically be used for each deoxygenation process, thereby avoiding the prior art poisoning and regeneration problems and assuring effective catalytic activity each time. On the other hand, a particulate carbonaceous support could be used in place of an alumina pellets support in a catalyst system such as described in European patent application No. 2,976 (as hereinbefore referred to) and the catalyst activity would be more reliably regenerable in that the absorption properties of the carbon prevent poisoning of the catalyst by hydrogen sulphide or other poison.

We believe that the final concentration of oxygen in an anaerobic jar is probably dependent on the initial rate of reaction and that this is limited by the rate of gas generation. Hence a more effective gas generating kit would improve the performance of the catalyst. Very low oxygen levels could be achieved by the inclusion with the catalyst of means for maintaining an elevated temperature. Such means could be a heating tape positioned within the flame trap.

Catalyst for use in the process according to the invention may be prepared by any method which results in a disposition of the catalytic metal or oxide thereof as a dispersion on and within the pores of the support. Conveniently, conventional chemical precipitation techniques are employed whereby to a solution of a salt or compound of the catalytic metal in a solvent also containing the support is added a reagent which causes the salt or compound to be converted to an insoluble compound or to the metallic state which is thus deposited on the support. The insoluble compound may be an oxide or hydroxide of the catalytic metal. The support may then be washed and dried and optionally heated to form the oxide of the catalytic metal, if desired, although a surface oxide may be formed spontaneously in air. The carbonaceous support may chemically assist in the deposition process to give a good dispersion of catalytic metal or oxide.

For the deposition of a catalytic metal comprising palladium, suitable salts are $Na_2Pd(NO_3)_4$ and $Na_2PdCl_4$ and a complex oxide may be deposited from an aqueous solution of either of these salts by addition of base.

Alternative methods of preparation include chemical vapour deposition, selective or unselective impregnation/absorption and the like, which methods may optionally be followed by a reduction step.

The invention also includes an anaerobic jar comprising a closeable and sealable container provided with means for the introduction of a pre-determinable quantity of hydrogen, means located within the jar for holding a catalyst, a catalyst held by the means, the catalyst comprising palladium, platinum, rhodium and/or iridium disposed on a porous carbonaceous support and located within a heat-conductive perforate container. Preferably the support comprises woven carbonaceous cloth.

Embodiments of the invention will now be described by way of example and with reference to the accompanying drawings, of which

Figure 1 is a graphical illustration showing the performance of a catalyst according to the invention compared with a prior art catalyst in a deoxygenation process carried out in an anaerobic jar;

Figure 2 is an exploded perspective view showing the component parts of a typical anaerobic jar of the type supplied by Oxoid Ltd. incorporating a catalyst according to the invention;

Figure 3 is a scanning electron micrograph (X 25) of a catalyst according to the invention and comprising Pd disposed on woven carbonaceous cloth; and

Figure 4 is a scanning electron micrograph (X 63) of the catalyst shown in Figure 3.

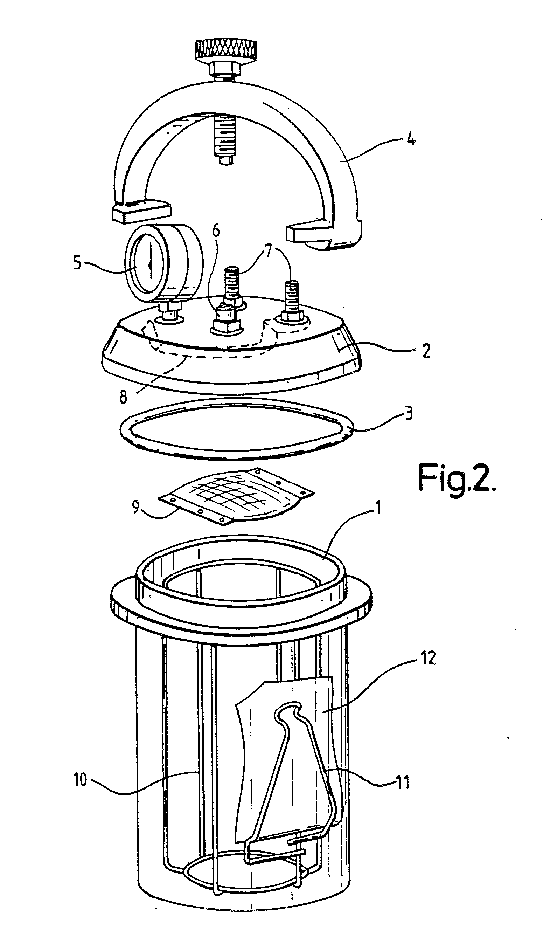

Referring to Figure 2, an anaerobic jar is shown and consists of flanged container 1 with lid 2 which is sealable on the container by means of sealing ring 3 and a clamp bar 4. The lid is fitted with pressure/vacuum gauge 5, pressure release valve 6 and "Schrader" valves 7 for introduction of gases from an external source, if desired. The underside of the lid carries a tongue 8 (shown in dotted outline) for retaining the catalyst, which is contained within metal gauze sachet 9 which acts as a flame trap. A framework 10 within the container for holding a stack of culture dishes has a clip 11 for holding a sachet 12 containing gas generating chemicals.

In use, sachet 12 is clipped in place on the framework, a catalyst is located in a gauze sachet 9 and retained by tongue 8, water is added to the generator chemicals, and the lid is closed and tightened to seal it in place. Hydrogen liberated by the generator chemicals react with oxygen on the catalyst to produce water vapour.

4

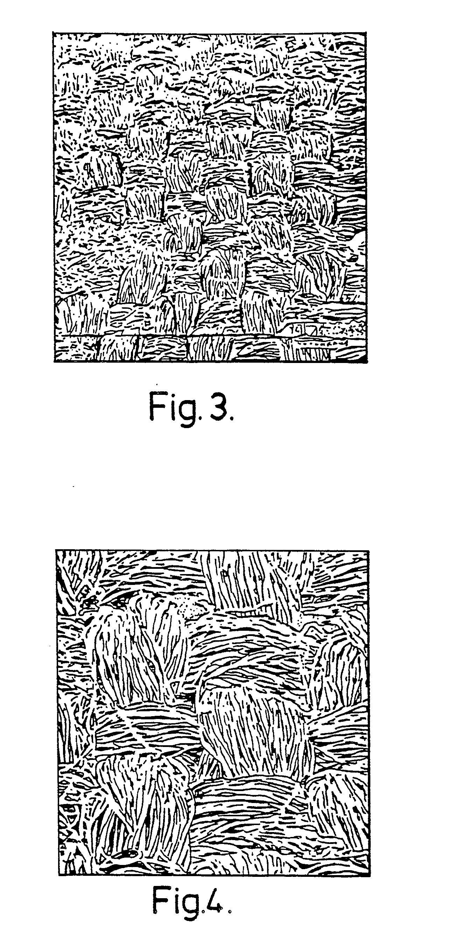

Figures 3 and 4 show in detail a sample of catalyst comprising palladium (1.78% by weight) disposed on a woven carbonaceous cloth ("C-tex") and suitable for inserting within the gauze sachet 9 of Figure 2.

Example 1

Catalysts consisting of palladium disposed on a carbonaceous cloth support were prepared in the laboratory by conventional techniques. In each case the support was an activated carbon cloth obtained from Siebe German & Co. Ltd. under the trade name "C-Tex". The surface area of this support was 880 $m^2g^{-1}$, as determined by the $N_2$/BET method. Squares of cloth measuring 10 cm × 10 cm were taken and an aqueous solution of a palladium salt was applied either from a dropper, by spraying or by immersion. The impregnated cloth was then dried following an optional hydrazine reduction step.

Application techniques and details of the resulting catalysts are given in Table 1 below. Oxidation states of palladium were determined by photoelectron spectroscopy, and an oxidation state of 0 indicates metal and 1 indicates oxide.

TABLE 1

| Catalyst No. | Application | Pd oxidation state | Pd (wt % of catalyst) | Pd (mg) | Metal surface area $(m^2g^{-1})$ |
|---|---|---|---|---|---|
| 1 | Dropper | — | 0.37 | 0.33 | 1.9 |
| 2 | Dropper | 0+1 | 1.32 | 1.19 | 9.7 |
| 3 | Immersion (2) | 0 | 1.90 | 1.76 | 16.4 |
| 4 | Dropper | 0+1 | 1.95 | 1.76 | 13.2 |
| 5 | Spray (3) | 1 | 2.43 | 2.19 | 23.8 |
| 6 | Spray | — | 1.56 | 2.81 | 16.2 |
| 7 | Immersion (1) | 0+1 | 6.09 | 5.48 | 53.8 |
| 8 | Immersion (2) | — | 7.57 | 6.81 | 69.0 |
| 9 | Dropper (4) | 0 | 3.01 | 2.71 | 11.8 |
| 10 | Dropper | 0 | 3.36 | 3.02 | 15.4 |

Notes to Table 1:
1. Immersion time 10 minutes
2. Immersion time 20 minutes
3. Size of cloth 10 cm × 10 cm
4. Reduced with hydrazine

The catalysts were evaluated by comparison in a deoxygenation process in an anaerobic jar with palladised alumina pellets prepared as described in European patent application No. 2,967. Details are given below with particular reference to catalyst No. 3.

The deoxygenation process was carried out in a standard anaerobic jar supplied by Oxoid Ltd, Basingstoke, Hampshire. An "Oxoid" gas generating kit comprising a sachet (similar to sachet 9 in Figure 2) containing tablets of sodium borohydride, tartaric acid and sodium bicarbonate was used to generate hydrogen and carbon dioxide inside the jar on the addition of water. The amount of chemicals in the kit is calculated to produce approximately 1800 ml. $H_2$ and 350 ml $CO_2$ at atmospheric pressure. The interior of the lid of the jar is provided with a tongue or clip for supporting a catalyst system, such as described in EP—A 2,967, contained within a metal gauze sachet.

We carried out two deoxygenation runs, each using a fresh gas generator kit, and using respectively a fresh prior art catalyst system and catalyst No. 3 disposed on a 2.54 cm square of carbonaceous cloth ("C-tex", ex Siebe-Gorman) cut from the 10 cm square prepared. The cloth weighed 90 mg, giving a palladium loading of 1.78% by weight.

For each run, the catalyst or catalyst system was inserted into the metal gauze flame trap constituted by the sachet 9 which was then located under the tongue 8 of the lid of the jar. Water was added to the gas generator kit 12, held by a spring clip 11 attached to the framework 10 within the jar for holding a stack of

5

culture dishes, and the lid 8 was secured in position. The pressure inside the jar was monitored by means of a pressure/vacuum gauge 5 mounted on the lid 8 and the course of the deoxygenation reaction was followed by withdrawing samples at intervals through one of the lid valves and measuring the oxygen concentation by gas chromatography.

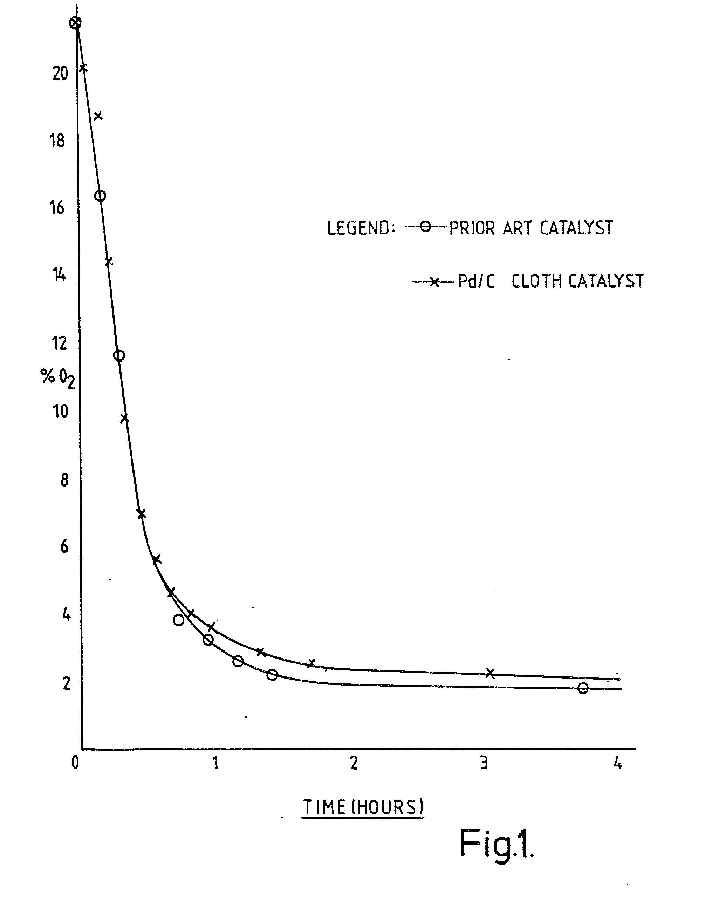

In each run the pressure inside the jar increased by about 3 psi in the first 15 minutes, held this pressure for about 15 minutes, and then gradually returned to atmospheric, the entire cycle taking about $1\frac{1}{2}$ hours. The decrease in oxygen concentration with time for each run is shown by the accompanying Figure 1, from which it is seen that the catalyst comprising palladium on carbonaceous cloth was as effective as a fresh (ie not regenerated) prior art alumina — supported catalyst in attaining an oxygen concentration of 6% (each taking $\frac{1}{2}$ hour) and in practical terms as effective as the alumina-supported catalyst in its oxygen removal by 2 hours (1.9% vs 2.4% remaining). The results shown in Figure 1 are not corrected for pressure or the presence of argon (which is included with the oxygen peak in chromatography), which correction would further lower the oxygen concentration.

Although the prior art catalyst pellets were held loose in and throughout the holes and folds of a rolled up metal foil net, to dissipate heat and to guard against the escape of catalyst "fines", no such precautions were taken for the carbonaceous cloth-supported catalyst. Despite this, the support effectively dissipated heat of reaction, no hot spots being formed, and there was no evidence of "fines" production or escape.

Of the other catalysts prepared in Example 1, catalyst No. 1 contained insufficient palladium to give an effective catalyst, and a substantial proportion of the palladium was poisoned by poisons present in the support. Catalyst No. 2 showed an improvement, achieving an oxygen level of 10% in 30 minutes and 4% in 2 hours, but was inferior to the prior art catalyst. Catalyst No. 5 achieved an oxygen level of 12% in 30 minutes and 3% in 3 hours, and exhibited an induction period before reaction started, thought to be due to preliminary *in situ* reduction of palladium oxide. Catalyst No. 6 was effectively equivalent to No. 3, in fact showing a marginal improvement between 1 and 3 hours. Catalyst Nos. 7 and 8 were better than No. 3 in the 30 minutes to 2 hours period. The pre-reduced catalyst No. 9 was superior to its non-reduced counterpart but was not as good as No. 3.

Further evaluations were carried out on catalysts prepared on a pilot plant. The carbon cloth was carbonised and activated viscose rayon obtained from Charcoal Cloth Ltd and having a designated "heat of wetting" specification of 40 to 60 joules/gm. The surface area was measured by $N_2$%BET and palladium was deposited to give palladium in the zero oxidation state.

Results are given in Table 2 below, compared with a catalyst prepared on the laboratory-scale.

TABLE 2

| Catalyst No. | Surface area of cloth $(m^2g^{-3})$ | Pd loading $(mg/6.45 cm^2)$ | Pd metal area $(m^2g^{-1}$ catalyst$)$ | $O_2$ content (I) after | |
|---|---|---|---|---|---|
| | | | | 90 mins | 120 mins |
| 11 (2) | 772 | 2.4 | 7.3 | 1.70 | 1.40 |
| 12 | 864 | 1.3 | — | 1.00 | 1.00 |
| 13 | 1019 | 2.6 | — | 5.00 | 4.46 |
| 14 | 866 | 1.9 | 8.7 | 2.12 | 1.61 |
| 15 | 1155 | 2.1 | 12.4 | 6.70 | 4.90 |
| 16 | 741 | 1.9 | 7.0 | 2.30 | 1.50 |

Notes to Table:
1. Uncorrected for argon content.
2. Laboratory scale preparation.

It can be seen from the columns depicting oxygen content that catalyst Nos. 11, 14 and 16 performed particularly well and achieved lower oxygen concentrations than both the prior art catalyst and catalyst No. 3 illustrated in Figure 1. It is noteworthy that catalysts 13 and 15, which were the both unacceptable in terms of performance were both prepared on high surface area cloth supports and were quenched by water.

**Claims**

1. A deoxygenation process comprising reacting together hydrogen and oxygen over a catalyst, wherein the catalyst comprises a catalytic metal selected from a palladium, platinum, rhodium and/or

6

iridium disposed on a porous, activated carbon, fibrous woven cloth support, having an average surface area in the range 200 to 1200 $m^2g^{-1}$.

2. A process according to claim 1, in which the cloth has an average surface area in the range 400 to 1000 $m^2g^{-1}$.

3. A process according to claim 1 or 2, in which the catalytic metal is in the form of a mixture or alloy with another platinum group metal or with a base metal as promoter or extender.

4. A process according to any of claims 1 to 3 in which the catalytic metal is present as a dispersion of crystallites having a metal area within the range of 3—100 $m^2g^{-1}$ of catalyst.

5. A process according to any preceding claim in which the loading of the catalytic metal is less than or equal to 5% by weight.

6. A process as claimed in claim 5 in which the catalytic metal is less than or equal to 2.5% by weight.

7. A process according to any one of claims 2 to 6, in which the catalytic metal comprises palladium and the support comprises activated carbon woven cloth, the palladium loading being in the range of 1.67 to 2.2% by weight inclusive.

8. A process according to any preceding claim in which the process is carried out in a sealed container.

9. A process according to any preceding claim, carried out in an anaerobic jar, in which the oxygen level is reduced to below 2% within 2 hours of sealing the jar, hydrogen being introduced into the jar to react with the oxygen to form water vapour, the catalytic reaction being initiated at room temperature and heat of reaction being dissipated by the support to avoid the formation of hot spots.

10. An anaerobic jar comprising a closeable and sealable container provided with means for the introduction of a pre-determinable quantity of hydrogen, a catalyst supported within the jar, the catalyst comprising palladium platinum, rhodium and/or iridium disposed on a porous, activated carbon, fibrous woven cloth support having an average surface area in the range 200 to 1200 $m^2g^{-1}$ and located within a heat-conductive perforate container.

## Patentansprüche

Desoxidationsverfahren bei welchem Wasserstoff und Sauerstoff in Anwesenheit eines Katalysators zur Reaktion gebracht werden, wobei der Katalysator ein katalytisches Metall enthält, das aus einem Palladium, Platin, Rhodium und/oder Iridium ausgewählt ist und auf einem porösen Aktivkohle-Träger aus faserigem Gewebe angeordnet ist, welches eine mittlere Oberfläche im Bereich von 200 bis 1200 $m^2g^{-1}$ aufweist.

2. Verfahren nach Anspruch 1, bei welchem das Gewebe eine mittlere Oberfläche im Bereich von 400 bis 1000 $m^2g^{-1}$ aufweist.

3. Verfahren nach Anspruch 1 oder 2, bei welchem das katalytische Metall in Form eines Gemisches oder einer Legierung mit einem anderen Metall aus der Platingruppe oder mit einem Grundmetall als Promotor oder Extender vorliegt.

4. Verfahren nach einem der Ansprüche 1 bis 3, bei welchem das katalytische Metall als Dispersion aus Kristalliten mit einer Metallfläche im Bereich von 3 bis 100 $m^2g^{-1}$ an Katalysator vorliegt.

5. Verfahren nach einem vorstehenden Anspruch, in welchem der Gehalt an katalytischem Metall gleich oder weniger als 5 Gew.-% ist.

6. Verfahren nach Anspruch 5, bei welchem das katalytische Metall gleich oder weniger als 2,5 Gew-% ausmacht.

7. Verfahren nach einem der Ansprüche 2 bis 6, bei welchem das katalytische Metall Palladium enthält und der Träger Aktivkohlegewebe enthält, wobei der Anteil an Palladium im Bereich von 1,67 bis 2,2 Gew.-% einschließlich beträgt.

8. Verfahren nach einem vorangehenden Anspruch, bei welchem das Verfahren in einem abgedichteten Behälter durchgeführt wird.

9. Verfahren nach einem vorangehenden Anspruch, durchgeführt in einem anaeroben Gefäß, in welchem der Sauerstoffpegel auf weniger als 2% innerhalb von 2 Stunden des Abdichtens des Gefäßes reduziert wird, wobei Wasserstoff in das Gefäß für die Reaktion mit dem Sauerstoff zur Bildung von Wasserdampf eingeführt wird, die katalytische Reaktion bei Raumtemperatur eingeleitet wird und Reaktionswärme von dem Träger zur Vermeidung der Bildung von Quellpunkten abgeleitet wird.

10. Anaerobes Gefäß, welches einen schließbaren und abdichtbaren Behälter aufweist, der mit einer Einrichtung zur Einführung einer vorbestimmten Wasserstoffmenge ausgestattet ist, wobei in dem Gefäß ein Katalysator abgestützt ist, welcher Palladium, Platin, Rhodium und/oder Iridium auf einem porösen, faserigen Aktivkohlegewebeträger mit einer mittleren Oberfläche im Bereich von 200 bis 1200 $m^2g^{-1}$ enthält und in einem wärmeleitenden perforierten Behälter angeordnet ist.

## Revendications

1. Procédé de désoxygénation comprenant la réaction ensemble de l'hydrogène et de l'oxygène sur un catalyseur où le catalyseur comprend un métal catalytique choisi parmi le palladium, platine, rhodium, et/ou iridium disposé sur un support de tissu fibreux, de carbone actif, poreux ayant une aire de surface moyenne dans l'intervalle de 200 à 1200 $m^2g^{-1}$.

2. Un procédé selon la revendication 1, dans lequel le tissu a une aire de surface moyenne dans l'intervalle de 400 à 1000 $m^2g^{-1}$.

3. Un procédé selon la revendication 1, ou 2, dans lequel le métal catalytique est sous forme d'un mélange ou alliage avec un autre métal du groupe platine ou avec un métal commun comme promoteur ou charge.

4. Un procédé selon l'une quelconque des revendications 1 à 3, dans lequel le métal catalytique est présent sous forme d'une dispersion de cristallites ayant une aire de métal dans l'intervalle de 3 à 100 $m^2g^{-1}$ de catalyseur.

5. Un procédé selon l'une quelconque des revendications précédentes dans lequel la charge de métal catalytique est inférieure ou égale à 5% en poids.

6. Un procédé selon la revendication 5 dans lequel le métal catalytique est inférieur ou égal à 2,5% en poids.

7. Un procédé selon l'une quelconque des revendications 2 à 6, dans lequel le métal catalytique comprend du palladium et le support comprend un tissu de carbone actif, la charge de palladium étant dans l'intervalle de 1,67 à 2,2% en poids inclusivement.

8. Un procédé selon l'une quelconque des revendications précédentes dans lesquelles le procédé est mis en oeuvre dans un conteneur étanche.

9. Un procédé selon l'une quelconque des revendications précédentes, mis en oeuvre dans un récipient anaérobie dans lequel le niveau d'oxygène est réduit jusqu'à moins de 2% en moins de deux heures après avoir rendu le récipient étanche, de l'hydrogène étant introduit dans le récipient pour réagir avec l'oxygène pour former la vapeur d'eau, la réaction catalytique étant initiée à température ambiante et la chaleur de réaction étant dissipée par le support pour éviter la formation de points chauds.

10. Un récipient anaérobie comprenant un conteneur pouvant être fermé et scellé pourvu de moyens pour l'introduction d'une quantité prédéterminable d'hydrogène, un catalyseur sur support dans le récipient, le catalyseur comprenant du palladium, platine, rhodium, et/ou iridium disposé sur un support de tissu fibreux de carbone actif poreux ayant une aire de surface moyenne dans l'intervalle de 200 à 1200 $m^2g^{-1}$ et disposé dans un conteneur perforé conducteur de la chaleur.

0 089 830

LEGEND: —⊖— PRIOR ART CATALYST

—×— Pd/C CLOTH CATALYST

TIME (HOURS)

Fig.1.

Fig.2.

Fig. 3.

Fig. 4.